(19) **Europäisches Patentamt**

**European Patent Office**

**Office européen des brevets**

(11) **EP 0 682 252 B1**

(12) **EUROPEAN PATENT SPECIFICATION**

(45) Date of publication and mention
of the grant of the patent:
**02.11.2000 Bulletin 2000/44**

(51) Int Cl.[7]: **G01N 33/49**, C12Q 1/02,
C12M 1/34

(21) Application number: **95302886.7**

(22) Date of filing: **27.04.1995**

(54) **Composite optical blood culture sensor**

Zusammengesetzter optischer Blutkultursensor

Détecteur optique composite d'une hémoculture

(84) Designated Contracting States:
**AT BE CH DE ES FR GB IT LI NL SE**

(30) Priority: **10.05.1994 US 241228**

(43) Date of publication of application:
**15.11.1995 Bulletin 1995/46**

(73) Proprietor: **Becton, Dickinson and Company
Franklin Lakes, New Jersey 07417-1880 (US)**

(72) Inventor: **Berndt, Klaus W.
Stewartstown, Pennsylvania 17363 (US)**

(74) Representative: **Ruffles, Graham Keith
MARKS & CLERK,
57-60 Lincoln's Inn Fields
London WC2A 3LS (GB)**

(56) References cited:
**EP-A- 0 590 775        GB-A- 2 132 348
US-A- 5 030 420**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

## BACKGROUND OF THE INVENTION

The present invention relates to a non-invasive method and apparatus for detecting biological activities in a fluid specimen, such as blood, urine or sputum, where the specimen and a culture medium are introduced into sealable containers and are exposed to conditions enabling a variety of metabolic, physical, and chemical changes to take place in the presence of microorganisms in the sample. The biological activity being detected by a variety of chemical sensors that are based on changes in fluorescence lifetime and/or intensity.

Usually, the presence of microorganisms such as bacteria in a patient's body fluid, particularly blood, is determined using blood culture vials. A small quantity of blood is injected through a sealing rubber septum into a sterile vial containing a culture medium. The vial is incubated at a temperature conducive to bacterial growth, e.g., 37°C, and monitored for such growth.

Common visual inspection involves monitoring the turbidity of the liquid suspension. Known instrumental methods detect changes in the $CO_2$ content in the headspace of the culture bottles, which is a metabolic by-product of the bacterial growth. Monitoring the $CO_2$ content can be accomplished by conventional methods, including radiochemical, infrared absorption at a $CO_2$ spectral line, or pressure/vacuum measurement. These methods, however, require invasive procedures which can result in cross-contamination between vials.

Recently, novel non-invasive methods have been developed which use chemical sensors inside a vial. Such sensors often respond to changes in the $CO_2$ concentration by changing their color or by changing their fluorescence intensity. The outputs from these sensors are based upon light intensity measurements. This means that errors may occur, particularly if the light sources used to excite the sensors, or the photodetectors used to monitor intensities, exhibit aging effects over time.

In known automated non-invasive blood culture systems, individual light sources, individual spectral excitation and emission filters, and individual photodetectors are arranged adjacent to each vial. Such arrangements result in certain station sensitivity variations from one vial to the next. Due to the fact that most known blood culture sensors generate only a moderate contrast ratio in the measured photocurrent during bacterial growth, extensive and time-consuming calibration procedures and sophisticated detection algorithms are required to operate these systems. Moreover, light sources, spectral filters, and photodetectors with extreme narrow specification tolerances must be utilized.

The disadvantage of such intensity-based sensor arrangements can be overcome by utilizing fluorescent sensors that change their fluorescence lifetime, wherein intensity measurement is replaced with time parameter measurement and intensity changes have no impact on the sensor output signal. Many chemical sensor materials are known that change their fluorescence lifetime with changing oxygen concentration, pH, carbon dioxide concentration, or other chemical parameters (see, e.g., G.B. Patent No. 2,132,348).

A change in sensor fluorescence lifetime is commonly monitored by applying a well-known phase shift method (see, e.g., U.S. Patent No. 5,030,420), wherein the excitation light is intensity-modulated. That method results in an intensity-modulated fluorescence emission that is phase-shifted relative to the excitation phase. Phase shift angle, $\theta$, being dependent on the fluorescence lifetime, $\tau$, according to the equation:

$$\tan \theta = \omega\tau \qquad (1)$$

where $\omega = 2\pi f$, is the circular light modulation frequency.

An inspection of equation (1) reveals that the phase shift method allows for maximum resolution, $d\theta/d\tau$, under the condition $\omega\tau = 1$. Unfortunately, almost all known pH- or carbon dioxide-sensitive fluorophores have decay times in the range 5 ns to 500 ps. In other words, light modulation frequencies, $f = 1/2\pi\tau$, in the range 32 MHz to 320 MHz would be required.

It is possible to accomplish light intensity modulation at such high frequencies, however, this would require acousto-optic or electro-optic modulators which are only efficient in combination with lasers. Moreover, detecting the modulated fluorescence light would require highly sensitive high-speed photodetectors, such as microchannel-plate photomultipliers, which are rather expensive. Consequently, all commercial automated blood culture systems are based on intensity monitoring, and none utilize time-resolved fluorescent carbon dioxide sensors.

EP-A590,775 provides a method and apparatus for detecting microorganisms in a large number of blood culture vials using more than one microorganism detection principle for each vial. Two of such possible detection means includes the use of a fluorescent carbon dioxide sensor and scattered photon migration. The apparatus performs a logic linkage of the results of all the detection principles applied. Therefore, an enhanced recovery and an improved accuracy in detecting microorganisms is achieved.

## SUMMARY OF THE INVENTION

The present invention overcomes problems identified in the art by providing a method and apparatus for reliably and non-invasively detecting biological activities in blood culture vials that do not have the fluorescence intensity limitations discussed above.

According to the present invention, a culture medium and blood specimen are introduced into a sealable glass vial having a headspace gas mixture such that a change in the gas mixture composition can be monitored by a chemically sensitive composite material in the vial. The chemically sensitive composite material comprises a mixture of a fluorophore and a chromophore. The fluorophore exhibits a long fluorescence decay time and a fluorescence intensity that depend on a first chemical parameter, such as oxygen concentration. The chromophore exhibits an optical transmission that depends on a second chemical parameter, such as carbon dioxide concentration, the optical transmission of the chromophore changing with the second chemical parameter either within the excitation or within the emission wavelength range of the fluorophore.

By illuminating the composite sensor matrix with intensity-modulated light, measuring the AC component and the DC component of the fluorescence photocurrent separately, and by processing these signals in a computer, a sensor output signal is produced that shows a significantly increased contrast ratio, compared with known optical blood culture sensors. In addition, the composite sensor allows for separation of the oxygen consumption effect and the carbon dioxide production effect, and requires only a relatively low light modulation frequency (150 kHz). Therefore, a low-cost light emitting diode (LED) can be used as the excitation source.

More particularly, the fluorophore and the chromophore are mixed into the same sensor matrix and are illuminated with intensity-modulated excitation light. The modulation frequency is chosen so that the condition $\omega\tau = 1$ holds for the fluorophore when the fluorescence lifetime has its minimum value. The fluorescence light emitted by the composite sensor is monitored using only one photodetector. The fluorescence photocurrent from the photodetector is split into its AC and DC components, that are measured separately. A sensor output signal is then generated by dividing the measured DC component by the calculated fluorescence modulation degree, which is equal to the AC/DC ratio of the components.

In an aerobic vial, bacterial growth causes at first a decrease in the oxygen concentration. This, in turn, results in an increase in the fluorescence intensity and in the fluorescence lifetime of the fluorophore. If the light modulation frequency for the excitation source is selected properly, the increase in lifetime causes a decrease in the fluorescence modulation degree. As soon as carbon dioxide is produced by the bacteria, the chromophore's optical transmission increases, which results in a further amplification in the emitted fluorescence intensity. In a sensor arrangement according to the present invention, the combined effects of oxygen consumption and carbon dioxide production generate a significantly higher change in the measured fluorescence photocurrent than in known blood culture sensors.

As mentioned above, the fluorescence modulation degree is also calculated. The modulation degree can be measured at very high precision because all artifacts due to light source aging, optical filter variations, vial displacement, and photodetector sensitivity changes are canceled out. Therefore, it is practical to utilize the modulation degree to calculate a final sensor output signal. This final signal is obtained by dividing the increasing DC component by the decreasing modulation degree. Due to the opposite trends of these two quantities, a further amplification effect results with regard to the final sensor output signal. Consequently, the sensor output signal shows a contrast ratio due to bacterial growth that is larger by almost two orders of magnitude as compared to known blood culture sensors. This allows for better detection, and the requirements with regard to part's tolerances are reduced.

In general, a time delay is observed between oxygen consumption and carbon dioxide production. Therefore, analyzing the final sensor output signal allows for the separation of the two effects. By taking into account the magnitude, speed, time of occurrence and relative time delay between the two mechanisms (oxygen and carbon dioxide), information regarding the microorganism species can be gained. Moreover, even if no time delay should occur, the two mechanisms can be resolved by analyzing the fluorescence modulation degree, as explained below.

These and other features, objects, benefits and advantages of the present invention will become more apparent upon reading the following detailed description of the preferred embodiments, along with the appended claims in conjunction with the drawings, wherein reference numerals identify corresponding components.

## DESCRIPTION OF THE DRAWINGS

Fig. 1 shows schematically a composite optical blood culture sensor arrangement according to the present invention;

Fig. 2 is a plot showing fluorescence intensity versus time for the fluorophore, in response to oxygen consumption by the microorganisms.

Fig. 3 is a plot showing frequency lifetime product, $\omega\tau$, versus time for the fluorophore, in response to oxygen consumption by the microorganisms;

Fig. 4 is a plot showing fluorescence intensity versus time in response to carbon dioxide production;

Fig. 5 is a plot showing modulation degree, AC/DC, of the fluorescence emission versus time in response to oxygen consumption and carbon dioxide production;

Fig. 6 is a plot showing fluorescence intensity versus time in response to oxygen consumption and delayed carbon dioxide production;

Fig. 7 is a plot showing fluorescence intensity divided by modulation degree versus time in response to oxygen consumption and delayed carbon dioxide production;

Fig. 8 is a plot showing the first derivative of the signal shown in Fig. 7;

Fig. 9 is a plot showing fluorescence intensity versus time, like the plot shown in Fig. 4, but for a weak carbon dioxide producer;

Fig. 10 is a plot showing fluorescence intensity divided by modulation degree versus time, like the plot shown in Fig. 7, but for a weak carbon dioxide producer.

Fig. 11 is a plot showing the first derivative of the signal shown in Fig. 10, for the weak carbon dioxide producer;

Fig. 12 is a plot showing standard spectral transmission characteristics for a pH/carbon dioxide-sensitive chromophore;

Fig. 13 is a plot showing modulation degree of the fluorescence emission for an oxygen sensor; and

Fig. 14 is a plot showing the first derivative of a growth curve and its characteristic features.

## DETAILED DESCRIPTION

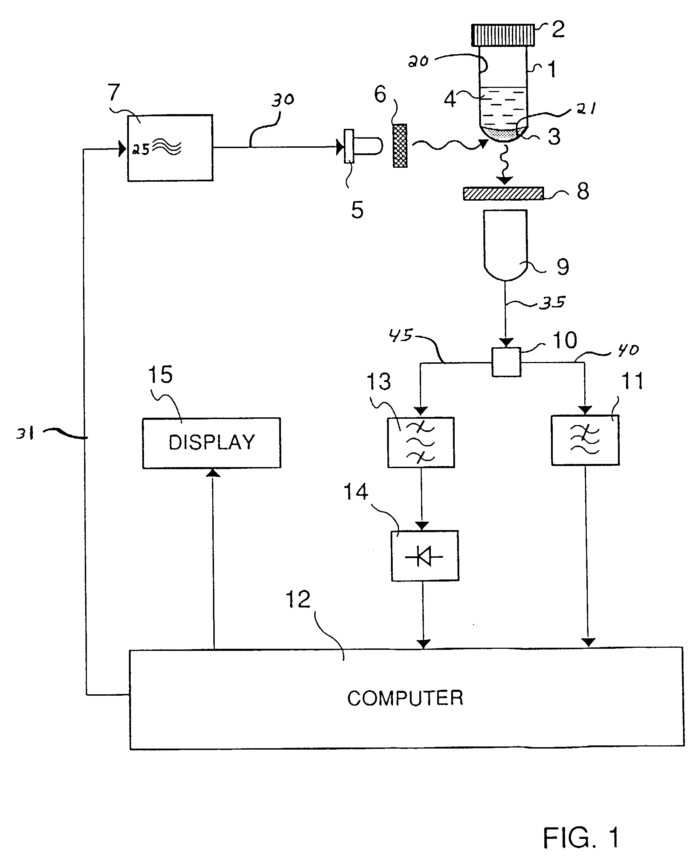

A preferred embodiment of a composite optical blood culture sensor arrangement embodying the principles and concepts of the invention is depicted schematically in Fig. 1. In this arrangement, a specimen and culture medium mixture 4 are introduced into an optically transparent container 1 that is sealed by a cap 2. A mixture of chemical sensor materials 3 is disposed to an inner wall 20 or an inner bottom surface 21 of container 1. The mixture 3 is illuminated by an excitation light source 5, preferably a blue LED 5, that is connected to an electronic signal source 7. Signal source 7 provides a DC bias and a high-frequency modulation voltage to light source 5 over a line 30, and is equipped with a power control input 25 connected by a line 31 to a computer 12.

Sensor material mixture 3 comprises a mixture of a fluorophore and a chromophore, wherein the fluorophore exhibits a long fluorescence decay time and a fluorescence intensity that depend on a first chemical parameter, such as oxygen concentration. The chromophore, however, exhibits an optical transmission that depends on a second chemical parameter, such as carbon dioxide concentration. The optical transmission of the chromophore changing with the second chemical parameter, either within the excitation or within the emission wavelength range of the fluorophore.

Fluorescence light reemerging from sensor material mixture 3 is detected by a photodetector 9, e. g., a photomultiplier. An emission filter 8 is arranged between mixture 3 and photodetector 9, and an excitation filter 6 is mounted in front of light source 5, to prevent excitation light from light source 5 reaching photodetector 9. Photodetector 9 generates an output signal on a line 35 that is fed to a power splitter 10. Power splitter 10 then generates two output signals, one of which is connected by a line 40 to the input of a low-pass filter 11 that is connected directly to computer 12. The other output signal of power splitter 10 is fed by a line 45 to the input of a band-pass filter 13 that is connected via a high-frequency voltmeter 14 to computer 12. Computer 12 is connected to a data display unit 15 to display information.

In operation, light source 5 illuminates sensor material mixture 3 with excitation light that is intensity-modulated at a circular modulation frequency, $\omega$, having a modulation degree, m. The emitted fluorescence intensity of sensor material mixture 3 can be described by:

$$F(t)=T(t)*F_0(t)[1+\frac{m}{\sqrt{1+(\omega\tau(t))^2}}\sin(\omega\tau-\theta(t))] \quad (2)$$

with T(t) being the carbon dioxide-dependent optical transmission of the chromophore, $F_0(t)$ being the oxygen-dependent average fluorescence intensity, and

$$\theta(t) = \arctan[\omega\tau(t)] \quad\quad\quad (3)$$

being the fluorescence phase shift relative to the excitation modulation phase. In equation (2), m represents the modulation degree of the excitation light, which can be as high as 1. For all plots that follow, a constant source modulation degree, m = 0.7, a very reasonable value, is assumed. The quantity $\tau(t)$ in equations (2) and (3) is fluorescence lifetime.

Average fluorescence intensity, $F_0(t)$, may depend on time, t, according to the expression:

$$F_0(t) = k_1 * h(t) \quad\quad\quad (4)$$

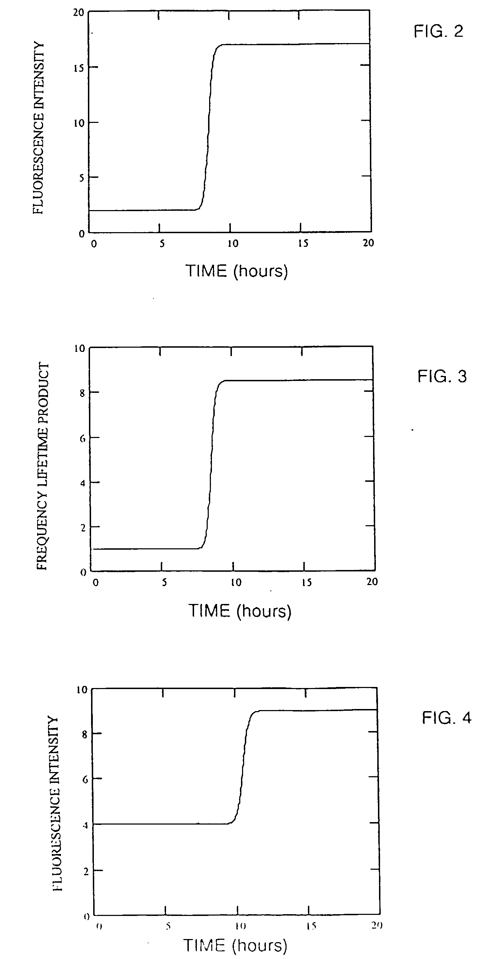

where $k_1$ is a constant, and h(t) is a time-dependent function that rises from a first, lower level to a second, higher level as a consequence of oxygen consumption during microorganism growth. Fig. 2 is a plot showing fluorescence intensity versus time for a fluorophore, in response to oxygen consumption by the microorgan-

isms.

Fluorescence lifetime $\tau(t)$, may depend on time, t, according to the expression:

$$\tau(t) = k_2 * h(t) \qquad (5)$$

where $k_2$ is a constant, and h(t) is the same time-dependent function that rises from a first, lower level to a second, higher level as a consequence of oxygen consumption during microorganism growth.

Modulation frequency, $\omega$, is chosen so that the condition $\omega\tau \approx 1$ holds for the fluorophore when it has its minimum $\tau$-value. Fig. 3 is a plot showing frequency lifetime product, $\omega\tau$, versus time for the fluorophore, in response to oxygen consumption by the microorganisms. The light modulation frequency is selected so that $\omega\tau = 1$ prior to oxygen consumption if the fluorescence lifetime is short.

Optical transmission of the chromophore, T(t), is also a time-dependent function that rises from a first, lower level to a second, higher level as a consequence of carbon dioxide production during microorganism growth. Fig. 4 is a plot showing the corresponding increase in the remitted fluorescence intensity versus time in response to carbon dioxide production. The carbon dioxide response exhibits a time delay relative to oxygen response.

In a composite optical blood culture sensor arrangement according to the present invention, the fluorescence photocurrent, I(t), is given by the expression:

$$I(t) = K * F(t) \qquad (6)$$

where K is a constant. Photocurrent I(t) is then split into AC and DC components that are measured separately, so that an AC/DC ratio can then be calculated within computer 12. Based on this, we obtain for the AC component:

$$AC = K*T(t)*F_0(t) \frac{m}{\sqrt{1+(\omega\tau(t))^2}} \qquad (7)$$

for the DC component:

$$DC = K*T(t)*F_0(t) \qquad (8)$$

and for the fluorescence modulation degree:

$$\frac{AC}{DC} = \frac{m}{\sqrt{1+(\omega\tau(t))^2}} \qquad (9)$$

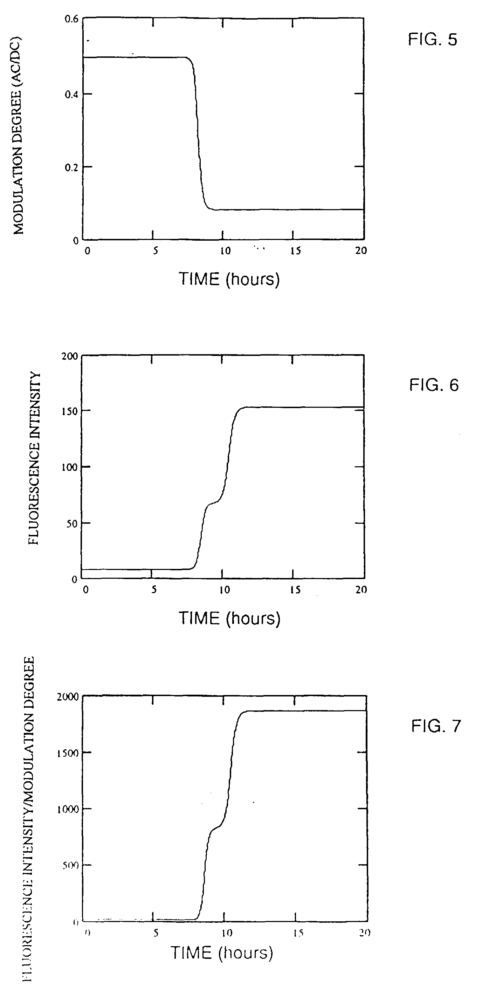

From equation (9), it can be seen that fluorescence modulation degree, AC/DC, does not change with a change in carbon dioxide concentration. This is illustrated in Fig. 5, where modulation degree, AC/DC, of the re-emitted fluorescence is plotted versus time, in response to oxygen consumption and carbon dioxide production. Equation (9) also shows that modulation degree, AC/DC, is independent of K, therefore, almost free from instrumental artifacts.

Fig. 6 is a plot of the DC signal, i.e., fluorescence intensity versus time in response to oxygen consumption and delayed carbon dioxide production. The contrast ratio due to the combined oxygen and carbon dioxide effect is 19.1, which is the product of the carbon dioxide contrast ratio of 2.25 and the oxygen contrast ratio of 8.5.

Fig. 7 is a plot showing fluorescence intensity, DC, divided by modulation degree, AC/DC, versus time in response to oxygen consumption and delayed carbon dioxide production. The plot in Fig. 7 represents a final sensor output signal that is displayed by computer 12 on display 15. It should be appreciated that the contrast ratio, relative to the contrast ratio in Fig. 6, is increased further from 19.1 to a value of 120.

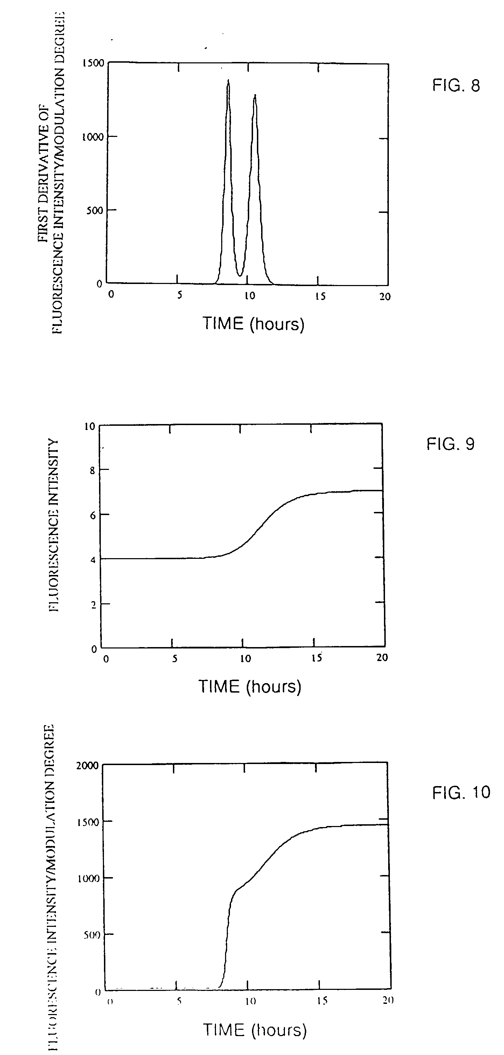

Fig. 8 is a plot showing a first derivative of the signal in Fig. 7, and illustrates that the effects of oxygen consumption and carbon dioxide production are clearly separated. In this case, both peaks exhibit comparable heights.

Applicant has also found that the effects of oxygen consumption and carbon dioxide production can also be resolved, if no time delay should occur. This is possible simply by analyzing the final sensor output signal and the modulation degree, AC/DC. While final sensor output responds to both effects, modulation degree responds only to the oxygen effect. Therefore, it is possible to separate each effect.

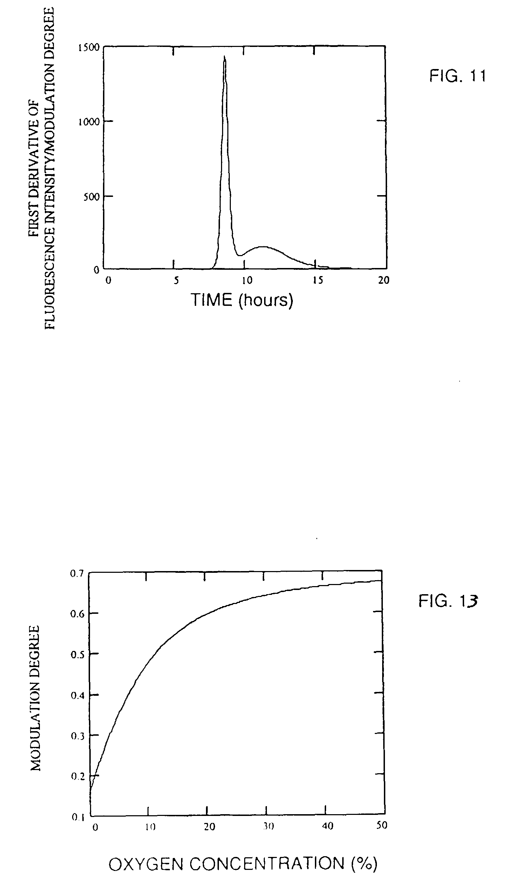

Fig. 9 corresponds to Fig. 4, but shows a plot of fluorescence intensity versus time for a weak carbon dioxide producer; Fig. 10 corresponds to Fig. 7, but shows the final sensor output signal expected for a weak carbon dioxide producer; and Fig. 11 corresponds to Fig. 8, but shows the differentiated signal expected for a weak carbon dioxide producer. Again, the effects of oxygen consumption and carbon dioxide production are clearly separated, but now the two peaks exhibit very different heights.

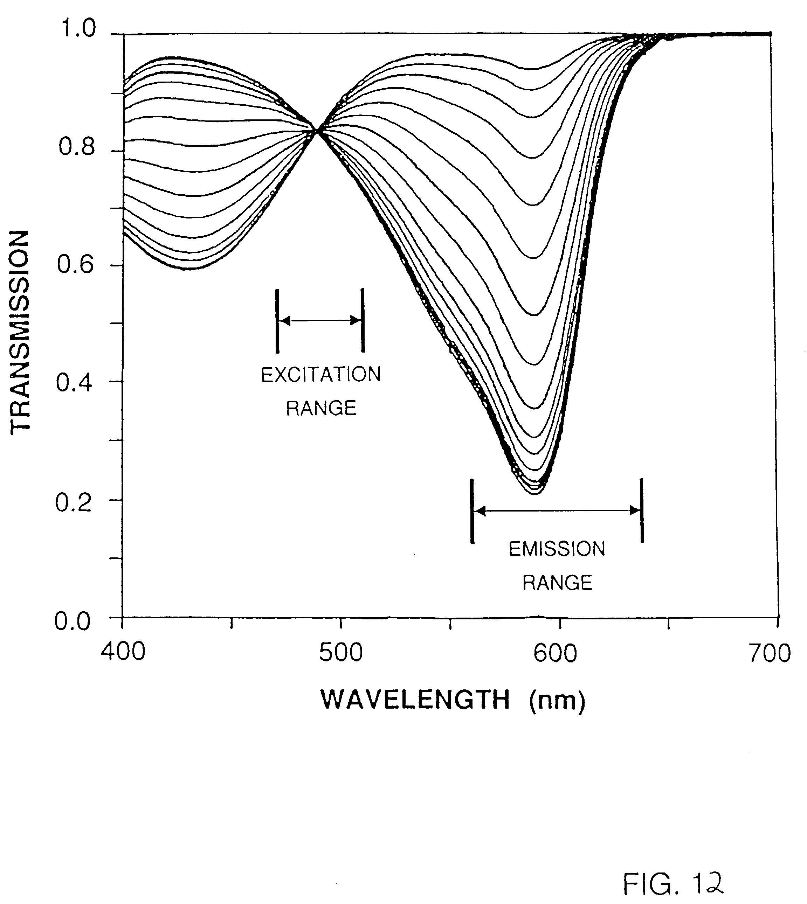

Fig. 12 is a plot showing a typical spectral transmission characteristic for a pH/carbon dioxide-sensitive chromophore. The family of curves shown in Fig. 12 illustrate the change in optical transmission with changing chemical input parameter, the indicated excitation range corresponding to the emission wavelength range of blue SiC light-emitting diodes. The indicated emission range corresponds to the emission wavelength range of available fluorescent oxygen sensors.

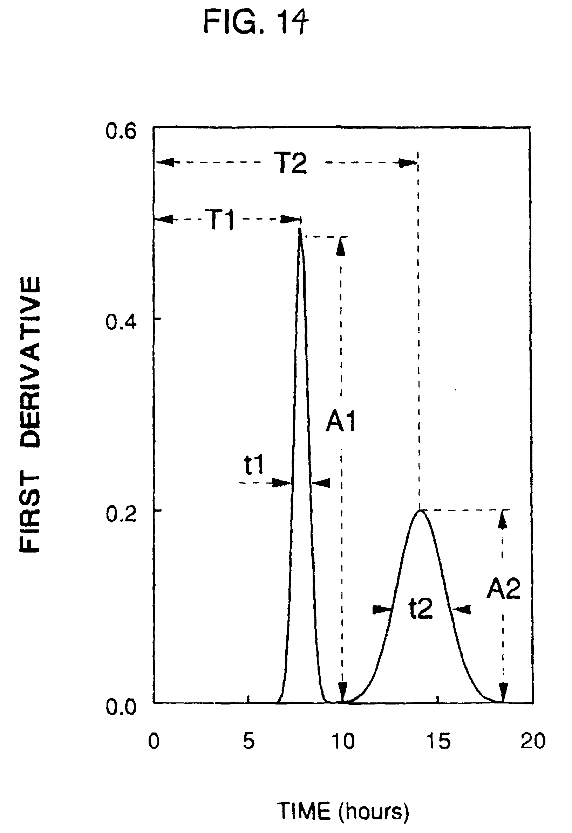

Fig. 13 shows the modulation degree, AC/DC, of a fluorescence emission for an oxygen sensor, the light modulation frequency having been set to 140 kHz.

In this case, the condition $\omega\tau = 1$ is fulfilled for an oxygen concentration of 11%. In doing so, changes in the initial high oxygen concentration have only a minor effect on the measured modulation degree. This is of advantage, in view of the required long shelf life of typical blood culture vials. In general, an optimization is possible by selecting a particular light modulation frequency.

Fig. 14 is a plot showing the first derivative of a growth curve and its characteristic features. In Fig. 14, T1 and T2 are the times of occurrence for the oxygen and the carbon dioxide effect. Parameters t1 and t2 indicate the duration for each of these effects and parameters Al and A2 are related to the strength of both effects. Applicant has found that by compiling a set of these six characteristic parameters for each sample vial and comparing the set with the corresponding data base that has been generated using known samples at an earlier time, a presumptive microorganism identification can be achieved.

It should be understood that the above-described embodiment is simply illustrative of an apparatus embodying the principles and concepts of the present invention. For example, the mixture of chemical sensor materials 3, shown in Fig. 1, disposed to the wall or bottom surface of container 1 could be replaced with a bi-layer structure having the fluorophore and chromophore contained in two adjacent layers rather than mixed together. The chromophore would be attached first to container 1 and the fluorophore would then be added as a second top layer. While this may be a little more expensive because of the two production steps required, the effect of changing chromophore transmission on the emitted fluorescence would be even stronger, since every fluorescence excitation and/or emission photon would have to traverse the full chromophore layer. In the mixed option, shown in Fig. 1, some photons never "meet" a chromophore molecule, but only meet a fluorophore molecule to generate a new fluorescence photon and therefore leave the sensor material without interacting with any chromophore molecule.

**Claims**

1. An apparatus for detecting microorganism growth comprising:

    a container comprising a culture medium, a blood specimen, and a headspace having a concentration of a gas;
    a chemically sensitive material in said container for detecting microorganism growth within said container when illuminated with an intensity-modulated light, said chemically sensitive material being comprised of a first material and a second material;

    wherein said first material is a fluorophore that exhibits a change in fluorescence decay time and fluorescence intensity in response to a change in a first chemical parameter of said gas; and wherein said second material is a chromophore that exhibits a change in optical transmission in response to a change in a second chemical parameter of said gas, whereby the change in fluorescence intensity emitted by said fluorophore is amplified by the optical transmission change of said chromophore;

    a light source for illuminating said chemically sensitive material in said container with the intensity-modulated light at a predetermined circular modulation frequency with a predetermined modulation degree;
    a photodetector for detecting fluorescence light reemerging from said chemically sensitive material;
    means for measuring an AC component and a DC component of the fluorescence photocurrent from said photodetector; and
    means for generating a sensor output signal based on the AC component and the DC component representing information as to whether or not microorganism growth is present within said container.

2. An apparatus according to Claim 1, wherein said first chemical parameter of said gas is oxygen concentration, such that said first material exhibits a change in fluorescence decay time and fluorescence intensity in response to the change in oxygen concentration of said gas.

3. An apparatus according to Claim 1, wherein said second chemical parameter of said gas is carbon dioxide concentration, such that said second material exhibits a change in optical transmission in response to the change in carbon dioxide concentration of said gas.

4. An apparatus according to Claim 1, further comprising:

    means for compiling a first set of signals corresponding to said fluorescence decay time and fluorescence intensity depending on said first chemical parameter of said gas;
    means for compiling a second set of signals corresponding to said optical transmission depending on said second chemical parameter of said gas; and
    means for comparing the first and second sets of signals with a corresponding predetermined data base of first and second sets of signals for known microorganisms to perform microorganism identification on a detected microorganism.

**Patentansprüche**

1. Vorrichtung zum Nachweis des Wachstums von Mikroorganismen, die aufweist:

   einen Behälter mit einem Kulturmedium, einer Blutprobe und einem Kopfraum mit einer Gaskonzentration;
   ein chemisch empfindliches Material in dem Behälter zum Nachweis von Mikroorganismenwachstum innerhalb des Behälters bei Beleuchtung mit intensitätsmoduliertem Licht, wobei sich das chemisch empfindliche Material aus einem ersten Material und einem zweiten Material zusammensetzt; wobei das erste Material ein Fluorophor ist, das als Reaktion auf eine Änderung eines ersten chemischen Parameters des Gases eine Änderung der Fluoreszenzabklingzeit und der Fluoreszenzintensität aufweist, und wobei das zweite Material ein Chromophor ist, das als Reaktion auf eine Änderung eines zweiten chemischen Parameters des Gases eine Änderung der Lichtdurchlässigkeit aufweist, wodurch die Änderung der durch das Fluorophor emittierten Fluoreszenzintensität durch die Lichtdurchlässigkeitsänderung des Chromophors verstärkt wird;
   eine Lichtquelle zum Beleuchten des chemisch empfindlichen Materials in dem Behälter mit dem intensitätsmodulierten Licht bei vorgegebener Modulationskreisfrequenz mit vorgegebenem Modulationsgrad;
   einen Photodetektor zum Erfassen von aus dem chemisch empfindlichen Material wiederaustretendem Fluoreszenzlicht;
   eine Einrichtung zum Messen einer Wechselstromkomponente und einer Gleichstromkomponente des Fluoreszenzphotostroms von dem Photodetektor; und
   eine Einrichtung zum Erzeugen eines auf der Wechselstromkomponente und der Gleichstromkomponente basierenden Sensorausgangssignals, das eine Information über das Vorhandensein oder Nichtvorhandensein von Mikroorganismenwachstum innerhalb des Behälters darstellt.

2. Vorrichtung nach Anspruch 1, wobei der erste chemische Parameter des Gases die Sauerstoffkonzentration ist, so daß das erste Material als Reaktion auf die Änderung der Sauerstoffkonzentration des Gases eine Änderung der Fluoreszenzabklingzeit aufweist.

3. Vorrichtung nach Anspruch 1, wobei der zweite chemische Parameter des Gases die Kohlendioxidkonzentration ist, so daß das zweite Material als Reaktion auf die Änderung der Kohlendioxidkonzentration des Gases eine Änderung der Lichtdurchlässigkeit aufweist.

4. Vorrichtung nach Anspruch 1, die ferner aufweist:

   eine Einrichtung zum Kompilieren bzw. Zusammenstellen einer ersten, der Fluoreszenzabklingzeit und der Fluoreszenzintensität entsprechenden Signalmenge in Abhängigkeit von dem ersten chemischen Parameter des Gases;
   eine Einrichtung zum Kompilieren einer zweiten, der Lichtdurchlässigkeit entsprechenden Signalmenge in Abhängigkeit von dem zweiten chemischen Parameter des Gases; und
   eine Einrichtung zum Vergleichen der ersten und zweiten Signalmengen mit einer entsprechenden vorgegebenen Datenbank von ersten und zweiten Signalmengen für bekannte Mikroorganismen zur Durchführung einer Mikroorganismen-Identifikation an einem erfaßten Mikroorganismus.

**Revendications**

1. Appareil pour détecter la croissance de micro-organisme, comprenant:

   un récipient contenant un milieu de culture, un échantillon de sang, et un espace de tête ayant une concentration d'un gaz;
   une matière chimiosensible dans ledit récipient pour détecter une croissance de micro-organisme dans ledit récipient lorsqu'elle est illuminée avec une lumière modulée en intensité, ladite matière chimiosensible étant composée d'une première matière et d'une seconde matière; dans laquelle ladite première matière est un fluorophore qui présente un changement du temps de décroissance de fluorescence et de l'intensité de fluorescence en réponse à un changement d'un premier paramètre chimique dudit gaz; et dans laquelle ladite seconde matière est un chromophore qui présente un changement de transmission optique en réponse à un changement d'un second paramètre chimique dudit gaz, de sorte que le changement d'intensité de fluorescence émise par ledit fluorophore est amplifié par le changement de transmission optique dudit chromophore;
   une source de lumière pour illuminer ladite matière chimiosensible dans ledit récipient avec la lumière modulée en intensité à une fréquence de modulation circulaire prédéterminée avec un degré de modulation prédéterminé;
   un photodétecteur pour détecter la lumière de fluorescence réémergeant de ladite matière chimiosensible;

un moyen pour mesurer une composante CA et une composante CC du photocourant de fluorescence dudit photodétecteur; et

un moyen pour générer un signal de sortie du détecteur reposant sur la composante CA et la composante CC indiquant si une croissance de micro-organisme est présente ou non dans ledit récipient.

2. Appareil selon la revendication 1, dans lequel ledit premier paramètre chimique dudit gaz est la concentration d'oxygène, de sorte que ladite première matière présente un changement du temps de décroissance de fluorescence et d'intensité de fluorescence en réponse au changement de concentration d'oxygène dudit gaz.

3. Appareil selon la revendication 1, dans lequel ledit second paramètre chimique dudit gaz est la concentration de dioxyde de carbone, de sorte que ladite seconde matière présente un changement de transmission optique en réponse au changement de concentration de dioxyde de carbone dudit gaz.

4. Appareil selon la revendication 1, comprenant en outre:

un moyen pour compiler un premier ensemble de signaux correspondant auxdits temps de décroissance de fluorescence et intensité de fluorescence en fonction dudit premier paramètre chimique dudit gaz;

un moyen pour compiler un second ensemble de signaux correspondant à ladite transmission optique en fonction dudit second paramètre chimique dudit gaz; et

un moyen pour comparer les premier et second ensembles de signaux avec une base de données prédéterminée correspondante des premier et second ensembles de signaux pour des micro-organismes connus pour effectuer l'identification de micro-organisme sur un micro-organisme détecté.

FIG. 1

FIG. 2

FIG. 3

FIG. 4

FIG. 5

FIG. 6

FIG. 7

FIG. 8

FIG. 9

FIG. 10

FIG. 11

FIG. 13

FIG. 12

# FIG. 14